(19) Europäisches Patentamt
European Patent Office
Office européen des brevets

(11) EP 2 771 034 B1

(12) **EUROPEAN PATENT SPECIFICATION**

(45) Date of publication and mention of the grant of the patent:
**08.08.2018 Bulletin 2018/32**

(21) Application number: **12783783.9**

(22) Date of filing: **25.10.2012**

(51) Int Cl.:
***A61K 45/06*** *(2006.01)* ***A61K 47/60*** *(2017.01)*

(86) International application number:
**PCT/US2012/061952**

(87) International publication number:
**WO 2013/063286 (02.05.2013 Gazette 2013/18)**

(54) **TOPOISOMERASE-I AND-II INHIBITORS FOR USE IN THE TREATMENT OF PATIENTS SUFFERING FROM CANCER**

TOPOISOMERASE-I UND -II HEMMER ZUR ANWENDUNG BEI DER BEHANDLUNG VON KREBSPATIENTEN

INHIBITEURS DE TOPOISOMERASE-I ET -II POUR UTILISATION DANS LE TRAITEMENT DE PATIENTS SOUFFRANT D'UN CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.10.2011 US 201161551138 P**

(43) Date of publication of application:
**03.09.2014 Bulletin 2014/36**

(73) Proprietor: **Nektar Therapeutics**
**San Francisco, CA 94158 (US)**

(72) Inventors:
- **HOCH, Ute**
  **San Francisco, CA 94110 (US)**
- **ELDON, Michael, A.**
  **Redwood City, CA 94062 (US)**
- **LEUNG, Abraham, C., F.**
  **Daly City, CA 94014 (US)**

(74) Representative: **Boult Wade Tennant**
**Verulam Gardens**
**70 Gray's Inn Road**
**London WC1X 8BT (GB)**

(56) References cited:
- **KRAUZE M.T. ET AL.: "Convection-enhanced delivery of nanoliposomal CPT-11 (irinotecan) and PEGYlated liposomal doxorubicin (Doxil) in rodent intracranial brain tumor xenografts", NEURO-ONCOLOGY, 1 October 2007 (2007-10-01), pages 393-403, XP002690547,**
- **PILLOT G. A. ET AL.: "Phase I study of pegylated liposomal doxorubicin (PLD) and irinotecan (I) in patients with solid tumors", JOURNAL OF CLINICAL ONCOLOGY, vol. 24, no. 18S, 1 June 2006 (2006-06-01) , page 12023, XP002690548, cited in the application**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 771 034 B1

## Description

### FIELD OF THE INVENTION

This invention relates to (among other things) the field of cancer chemotherapy and involves long-acting topoisomerase I inhibitors for use in the treatment of an individual suffering from a cancer by administering to the patient a long acting topoisomerase I inhibitor and a long acting topoisomerase II inhibitor.

### BACKGROUND OF THE INVENTION

Topoisomerase I and topoisomerase II are enzymes that play important and critical roles in cellular proliferation. As such, by inhibiting these enzymes, highly proliferative cells are preferentially targeted and unable to propagate. Thus, these enzymes are highly attractive targets for chemotherapeutic agents, especially in human cancers.

With respect to topoisomerase I, this class of enzyme catalyzes the uncoiling of DNA during replication and transcription. See Pommier et al. (1998) Biochim. Biophys. Acta. 1400(1-3):83-105 and Wang (1996) Annu. Rev. Biochem. 65:635-92).

The activity of topoisomerase I is regulated by phosphorylation, primarily on serine residues [Turman et al. (1993) Biochem. Med. Metab. Biol. 50(2):210-25; Coderoni et al. (1990) Int. J. Biochem. 22(7):737-46; Kaiserman et al. (1988) Biochemistry 27(9):3216-22; Samuels et al. (1992) J. Biol. Chem. 267(16): 1156-62)], and appears to be necessary for the initial complex formation between the enzyme and DNA (Coderoni et al. (1990) Int. J. Biochem. 22(7):737-46).

With respect to topoisomerase II, this class of enzyme acts by creating breaks in DNA, thereby allowing DNA strands to unravel and separate. Unlike topoisomerase I enzymes, topoisomerase II enzymes use the hydrolysis of adenosine-5' triphosphate.

Previously, it has been proposed to inhibit different steps of the DNA replication pathway via administration of a topoisomerase I inhibitor and a topoisomerase II inhibitor. See, for example, Pillot et al. (2006) Journal of Clinical Oncology 24(18S): 12023. Even if such regimens are feasible, administering such potent inhibitors together has drawbacks, including the risk of severe side effects.

Krauze et al. (1 October 2007; Neuro-oncology; 393-404) describe rodents being treated with PEGylated liposomal doxorubicin (Doxil) containing the topoisomerase II inhibitor doxorubicin in combination with liposomes containing the topoisomerase I inhibitor CPT-11.

Thus, there remains a need to provide (among other things) treatment regimens where combination strategies show tolerability along with acceptable efficacy.
The present invention seeks to address these and other needs in the art.

### SUMMARY OF THE INVENTION

The present invention provides a long-acting topoisomerase I inhibitor for use in a method comprising the steps of: (a) administering to a patient a topoisomerase II-inhibiting amount of a long-acting topoisomerase II inhibitor; and (b) administering to the patient a topoisomerase I-inhibiting amount of a long-acting topoisomerase I inhibitor, wherein the long-acting topoisomerase I inhibitor encompasses the following structure

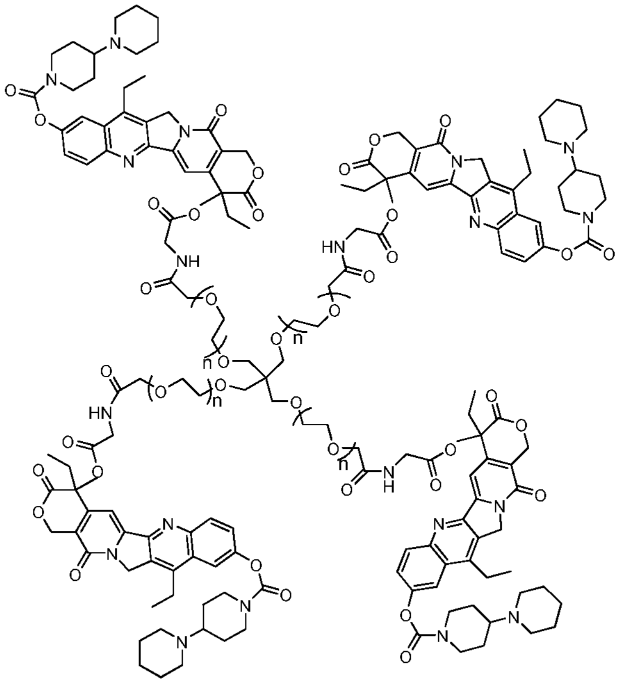

wherein each n is an integer ranging from 40 to 500, and pharmaceutically acceptable salts thereof, and wherein the long-acting topoisomerase II inhibitor is a topoisomerase II inhibitor which is released from its formulation over at least ten days.

By way of clarity, with regard to the sequence of steps in accordance with this method, unless otherwise indicated, the method is not limited to the sequence of steps and step (a) can be performed before, after or simultaneously with performing step (b).

Additional embodiments of the invention are set forth in the following description and claims.

## BRIEF DESCRIPTION OF THE DRAWINGS

**FIG. 1** is a scattergram plot of individual time-to-endpoint ("TTE") values obtained in connection with Example 2.

**FIG. 2** shows the median tumor growth curves (upper panel) and Kaplan-Meier plots (lower panel) for all groups tested in connection with Example 2.

**FIG. 3** presents the percent group mean body weight changes from day 1 for all groups tested in connection with Example 2.

## DETAILED DESCRIPTION OF THE INVENTION

As used in this specification, the singular forms "a," "an," and "the" include plural referents unless the context clearly dictates otherwise.

In describing and claiming the present invention, the following terminology will be used in accordance with the definitions described below.

"Water soluble, non-peptidic polymer" refers to a polymer that is at least 35% (by weight) soluble, preferably greater than 70% (by weight), and more preferably greater than 95% (by weight) soluble, in water at room temperature. Typically, an unfiltered aqueous preparation of a "water-soluble" polymer transmits at least 75%, more preferably at least 95%, of the amount of light transmitted by the same solution after filtering. It is most preferred, however, that the water-soluble polymer is at least 95% (by weight) soluble in water or completely soluble in water. With respect to being "non-peptidic," a polymer is non-peptidic when it has less than 35% (by weight) of amino acid residues.

The terms "monomer," "monomeric subunit" and "monomeric unit" are used interchangeably herein and refer to one of the basic structural units of a polymer. In the case of a homo-polymer, a single repeating structural unit forms the polymer. In the case of a co-polymer, two or more structural units are repeated -- either in a pattern or randomly --

to form the polymer. Preferred polymers used in connection with present the invention are homo-polymers. The water-soluble, non-peptidic polymer comprises one or more monomers serially attached to form a chain of monomers.

"PEG" or "polyethylene glycol," as used herein, is meant to encompass any water-soluble poly(ethylene oxide). Unless otherwise indicated, a "PEG polymer" or a polyethylene glycol is one in which substantially all (preferably all) monomeric subunits are ethylene oxide subunits, though, the polymer may contain distinct end capping moieties or functional groups, e.g., for conjugation. PEG polymers for use in the present invention will comprise one of the two following structures: "$-(CH_2CH_2O)_n-$" or "$-(CH_2CH_2O)_{n-1}CH_2CH_2-$," depending upon whether or not the terminal oxygen(s) has been displaced, e.g., during a synthetic transformation. As stated above, for the PEG polymers, the variable (n) ranges from about 3 to 4000, and the terminal groups and architecture of the overall PEG can vary.

"Branched," in reference to the geometry or overall structure of a polymer, refers to a polymer having two or more polymer "arms" extending from a branch point.

A "physiologically cleavable" or "hydrolyzable" or "degradable" bond is a relatively labile bond that reacts with water (i.e., is hydrolyzed) under physiological conditions. The tendency of a bond to hydrolyze in water may depend not only on the general type of linkage connecting two atoms within a given molecule but also on the substituents attached to these atoms. Appropriate hydrolytically unstable or weak linkages include but are not limited to carboxylate ester, phosphate ester, anhydrides, acetals, ketals, acyloxyalkyl ether, imines, orthoesters, peptides, oligonucleotides, thioesters, and carbonates.

An "enzymatically degradable linkage" means a linkage that is subject to degradation by one or more enzymes.

A "stable" linkage or bond refers to a chemical bond that is substantially stable in water, that is to say, does not undergo hydrolysis under physiological conditions to any appreciable extent over an extended period of time. Examples of hydrolytically stable linkages include but are not limited to the following: carbon-carbon bonds (e.g., in aliphatic chains), ethers, amides, urethanes, amines, and the like. Generally, a stable linkage is one that exhibits a rate of hydrolysis of less than about 1-2% per day under physiological conditions. Hydrolysis rates of representative chemical bonds can be found in most standard chemistry textbooks.

"Substantially" or "essentially" means nearly totally or completely, for instance, 95% or greater, more preferably 97% or greater, still more preferably 98% or greater, even more preferably 99% or greater, yet still more preferably 99.9% or greater, with 99.99% or greater being most preferred of some given quantity.

"Pharmaceutically acceptable excipient" or "pharmaceutically acceptable carrier" refers to a component that may be included in the compositions of the invention causes no significant adverse toxicological effects to a patient.

The term "patient," refers to a living organism suffering from or prone to a condition that can be prevented or treated by administration of a compound of the invention as described herein, and includes both humans and animals.

As indicated above, the present invention is directed to (among other things) a method comprising the steps of (a) administering to a patient a topoisomerase II-inhibiting amount of a long-acting topoisomerase II inhibitor; and (b) administering to the patient a topoisomerase I-inhibiting amount of a long-acting topoisomerase I inhibitor. With respect to administering steps (a) and (b), these administering steps can be performed in either order (as well as simultaneously) and the invention is not limited in this regard. In one or more embodiments of the invention, administering step (a) will be carried out before step administering (b). In one or more embodiments of the invention, administering step (b) will be carried out before administering step (a). In one or more embodiments, both administering steps (a) and (b) will be carried out simultaneously.

In those instances where administering to a patient a topoisomerase II-inhibiting amount of a long-acting topoisomerase II inhibitor occurs prior to administering to the patient a topoisomerase I-inhibiting amount of a long-acting topoisomerase I inhibitor, the amount of time that passes after administering the long acting topoisomerase II inhibitor prior to administering the long acting topoisomerase I inhibitor is preferably within one of the following ranges: from about one minute to about sixty days; from about one minute to about thirty days; from about one minute to about 21 days; from about ten minutes to about 21 days; from about twenty minutes to about 21 days; from about thirty minutes to about 21 days; from about forty minutes to about 21 days; from about sixty minutes to about 21 days; from about two hours to about 21 days; from about four hours to about 21 days; from about six hours to about 21 days; from about eight hours to about 21 days; from about ten hours to about 21 days; from about twelve hours to about 21 days; from about one day to about 21 days; from about two days to about 21 days; from about three days to about 21 days; from about four days to about 21 days; from about five days to about 21 days; from about six days to about 21 days; from about seven days to about 21 days; from about eight days to about 21 days; from about nine days to about 21 days; from about ten days to about 21 days; from about 14 days to about 21 days; from about one minute to about 14 days; from about ten minutes to about 14 days; from about twenty minutes to about 14 days; from about thirty minutes to about 14 days; from about forty minutes to about 14 days; from about sixty minutes to about 14 days; from about two hours to about 14 days; from about four hours to about 14 days; from about six hours to about 14 days; from about eight hours to about 14 days; from about ten hours to about 14 days; from about twelve hours to about 14 days; from about one day to about 14 days; from about two days to about 14 days; from about three days to about 14 days; from about four days to about 14 days; from about five days to about 14 days; from about six days to about 14 days; from about seven days to about 14 days; from

about eight days to about 14 days; from about nine days to about 14 days; from about ten days to about 14 days; from about one minute to about 8 days; from about ten minutes to about 8 days; from about twenty minutes to about 8 days; from about thirty minutes to about 8 days; from about forty minutes to about 8 days; from about sixty minutes to about 8 days; from about two hours to about 8 days; from about four hours to about 8 days; from about six hours to about 8 days; from about eight hours to about 8 days; from about ten hours to about 8 days; from about twelve hours to about 8 days; from about one day to about 8 days; from about two days to about 8 days; from about three days to about 8 days; from about four days to about 8 days; from about five days to about 8 days; from about six days to about 8 days; from six days to about 15 days; from about 13 days to about 22 days; from about 20 days to about 22 days; from about 20 days to about 29 days; from about 27 days to about 30 days; from about 27 days to about 45 days; and from about 45 days to about 75 days.

In those instances where administering to a patient a topoisomerase I-inhibiting amount of a long-acting topoisomerase I inhibitor occurs prior to administering to the patient a topoisomerase II-inhibiting amount of a long-acting topoisomerase II inhibitor, the amount of time that passes after administering the long acting topoisomerase I inhibitor prior to administering the long acting topoisomerase II inhibitor is preferably within one of the following ranges: from about one minute to about sixty days; from about one minute to about thirty days; from about one minute to about 21 days; from about ten minutes to about 21 days; from about twenty minutes to about 21 days; from about thirty minutes to about 21 days; from about forty minutes to about 21 days; from about sixty minutes to about 21 days; from about two hours to about 21 days; from about four hours to about 21 days; from about six hours to about 21 days; from about eight hours to about 21 days; from about ten hours to about 21 days; from about twelve hours to about 21 days; from about one day to about 21 days; from about two days to about 21 days; from about three days to about 21 days; from about four days to about 21 days; from about five days to about 21 days; from about six days to about 21 days; from about seven days to about 21 days; from about eight days to about 21 days; from about nine days to about 21 days; from about ten days to about 21 days; from about 14 days to about 21 days; from about one minute to about 14 days; from about ten minutes to about 14 days; from about twenty minutes to about 14 days; from about thirty minutes to about 14 days; from about forty minutes to about 14 days; from about sixty minutes to about 14 days; from about two hours to about 14 days; from about four hours to about 14 days; from about six hours to about 14 days; from about eight hours to about 14 days; from about ten hours to about 14 days; from about twelve hours to about 14 days; from about one day to about 14 days; from about two days to about 14 days; from about three days to about 14 days; from about four days to about 14 days; from about five days to about 14 days; from about six days to about 14 days; from about seven days to about 14 days; from about eight days to about 14 days; from about nine days to about 14 days; from about ten days to about 14 days; from about one minute to about 8 days; from about ten minutes to about 8 days; from about twenty minutes to about 8 days; from about thirty minutes to about 8 days; from about forty minutes to about 8 days; from about sixty minutes to about 8 days; from about two hours to about 8 days; from about four hours to about 8 days; from about six hours to about 8 days; from about eight hours to about 8 days; from about ten hours to about 8 days; from about twelve hours to about 8 days; from about one day to about 8 days; from about two days to about 8 days; from about three days to about 8 days; from about four days to about 8 days; from about five days to about 8 days; from about six days to about 8 days; from six days to about 15 days; from about 13 days to about 22 days; from about 20 days to about 22 days; from about 20 days to about 29 days; from about 27 days to about 30 days; from about 27 days to about 45 days; and from about 45 days to about 75 days.

The topoisomerase I inhibitor of the invention is long acting. A topoisomerase I inhibitor is long acting when the effective half life of the topoisomerase inhibitor satisfies one or more of the following ranges: from about 5 days to about 60 days; from about 9 days to about 60 days; from about 13 days to about 60 days; from about 21 days to about 60 days; from about 28 days to about 60 days; from about 35 days to about 60 days; from about 42 days to about 60; and from about 49 days to about 60 days.

With regard to the effective half life of a drug such as a topoisomerase I inhibitor, some topoisomerase I inhibitors metabolize into SN-38, which can be primarily responsible for the inhibitory activity of topoisomerase I. As such, those topoisomerase I inhibitors that metabolize into SN-38 often describe their half-lives in terms of the elimination of SN-38 (rather than on the elimination of the topoisomerase I inhibitor itself). Thus, as used herein, the "effective" half-life of a topoisomerase I inhibitor drug is the half-life of the entity -- whether the originally administered drug or a metabolite of the originally administered drug -- most responsible for the inhibitory activity of topoisomerase I. By way of example, the literature reports the effective half-life of irinotecan (based on the elimination of SN-38) is about two days, while the effective half-life (again, based on the elimination of SN-38) of a topoisomerase-inhibitor polymer conjugate is about fifty days. See Kehrer et al. (2000) Clin. Can. Res. 6:3451-3458 and Garcia et al. (2011) Abstract 5047, Poster #15C presented at the 2011 American Society of Clinical Oncology Annual Meeting, respectively.

The long acting topoisomerase I inhibitors encompass the following structure:

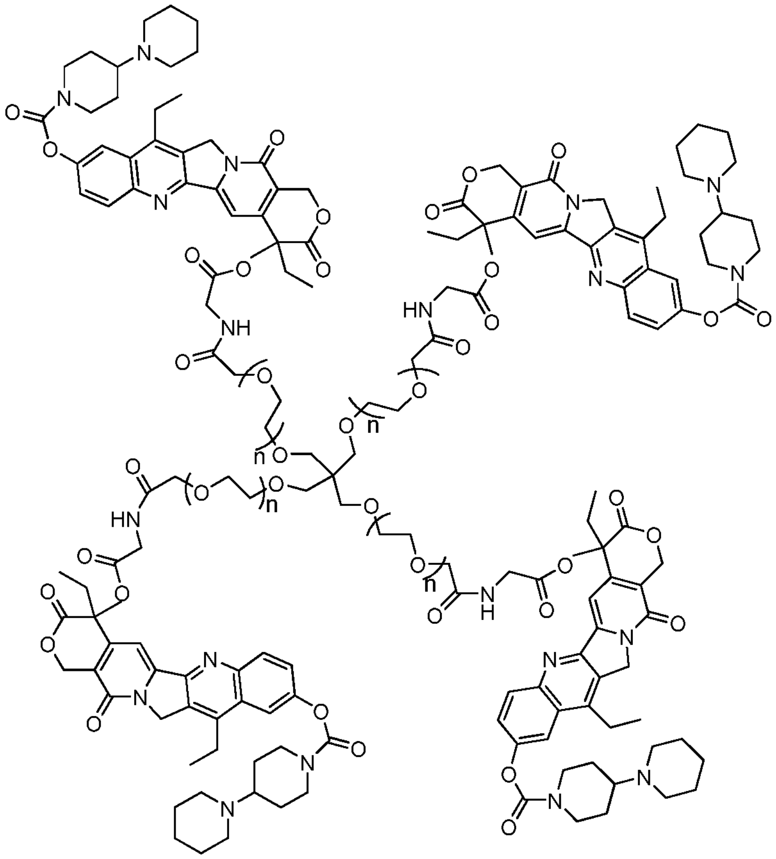

wherein each n is an integer ranging from 40 to about 500 (e.g., about 113 and about 226), and pharmaceutically acceptable salts (included mixed salts) thereof. The above and other compounds are described in U.S. Patent No. 7,744,861, and are considered "pentaerythritol-based multi-arm polymer conjugates of irinotecan" or a "PBMAPCI."

The method described herein involves the administration of a long acting topoisomerase II inhibitor. In this regard, the invention is not limited to any specific topoisomerase II inhibitor so long as the topoisomerase inhibitor is long acting. A topoisomerase inhibitor is long acting when the topoisomerase inhibitor is released from its formulation over at least ten days. By way of an exemplary long acting topoisomerase II inhibitor is doxorubicin HCL liposome injection (available under the DOXIL® brand from Janssen Biotech, Inc., Horsham PA). In this regard, the half-life for release of doxorubicin from liposomes *in vivo* has been reported as 315 hours for doxorubicin HCL liposome injection. See, for example, Laginha et al. (2005) Clin. Can. Res. 11(19):6944-6949.

Assays for determining whether a given compound can act as a long acting topoisomerase I inhibitor or a long acting topoisomerase II inhibitor can be determined through routing experimentation by one of ordinary skill in the art.

In accordance with the method described herein, the long acting topoisomerase II inhibitor is administered to a patient in a topoisomerase II-inhibiting amount. One of ordinary skill in the art can determine how much a given topoisomerase II inhibitor is sufficient to provide clinically relevant inhibition of topoisomerase II. For example, one of ordinary skill in the art can refer to the literature and/or administer a series of increasing amounts the topoisomerase inhibitor and determine which amount or amounts provide clinically relevant inhibition of topoisomerase II.

In one or more instances, however, the topoisomerase II-inhibiting amount (particularly with respect to doxorubicin HCL liposome injection available under the DOXIL® brand from Janssen Biotech, Inc., Horsham PA) is an amount encompassed by one or more of the following ranges: from about 1 mg/m$^2$ to about 1000 mg/m$^2$ of body surface; from about 2 mg/m$^2$ to about 900 mg/m$^2$ of body surface; from about 3 mg/m$^2$ to about 800 mg/m$^2$ of body surface; from about 4 mg/m$^2$ to about 700 mg/m$^2$ of body surface; from about 5 mg/m$^2$ to about 600 mg/m$^2$ of body surface; from about 6 mg/m$^2$ to about 550 mg/m$^2$ of body surface; from about 7 mg/m$^2$ to about 500 mg/m$^2$ of body surface; from about 8 mg/m$^2$ to about 450 mg/m$^2$ of body surface; from about 9 mg/m$^2$ to about 400 mg/m$^2$ of body surface; from about 5 mg/m$^2$ to about 200 mg/m$^2$ of body surface; from about 2 mg/m$^2$ to about 150 mg/m$^2$ of body surface; from about 5 mg/m$^2$ to about 100 mg/m$^2$ of body surface; from about 10 mg/m$^2$ to about 100 mg/m$^2$ of body surface; and from about 10 mg/m$^2$ to about 60 mg/m$^2$ of body surface.

In accordance with the method described herein, the long acting topoisomerase I inhibitor is administered to a patient in a topoisomerase I-inhibiting amount. One of ordinary skill in the art can determine how much a given

topoisomerase I inhibitor is sufficient to provide clinically relevant inhibition of topoisomerase I. For example, one of ordinary skill in the art can refer to the literature and/or administer a series of increasing amounts the topoisomerase inhibitor and determine which amount or amounts provide clinically relevant inhibition of topoisomerase I.

In one or more instances, however, the topoisomerase I-inhibiting amount (particularly with respect to a pentaerythritol-based multi-arm polymer conjugate of irinotecan) is an amount encompassed by one or more of the following ranges: from about 1 mg/m$^2$ to about 1000 mg/m$^2$ of body surface; from about 2 mg/m$^2$ to about 900 mg/m$^2$ of body surface; from about 3 mg/m$^2$ to about 800 mg/m$^2$ of body surface; from about 4 mg/m$^2$ to about 700 mg/m$^2$ of body surface; from about 5 mg/m$^2$ to about 600 mg/m$^2$ of body surface; from about 6 mg/m$^2$ to about 550 mg/m$^2$ of body surface; from about 7 mg/m$^2$ to about 500 mg/m$^2$ of body surface; from about 8 mg/m$^2$ to about 450 mg/m$^2$ of body surface; from about 9 mg/m$^2$ to about 400 mg/m$^2$ of body surface; from about 10 mg/m$^2$ to about 350 mg/m$^2$ of body surface; from about 20 mg/m$^2$ to about 200 mg/m$^2$ of body surface; from about 30 mg/m$^2$ to about 200 mg/m$^2$ of body surface; from about 40 mg/m$^2$ to about 270 mg/m$^2$ of body surface; and from about 50 mg/m$^2$ to about 240 mg/m$^2$ of body surface.

The actual dose to be administered will vary depend upon the age, weight, and general condition of the subject as well as the severity of the condition being treated, the judgment of the health care professional, and conjugate being administered.

The unit dosage of any given long acting topoisomerase I and long acting topoisomerase II inhibitor can be administered in a variety of dosing schedules depending on the judgment of the clinician, needs of the patient, and so forth. The specific dosing schedule will be known by those of ordinary skill in the art or can be determined experimentally using routine methods. Exemplary dosing schedules include, without limitation, administration five times a day, four times a day, three times a day, twice daily, once daily, three times weekly, twice weekly, once weekly, twice monthly, once monthly, and any combination thereof. Once the clinical endpoint has been achieved, dosing of the composition is halted.

The invention provides a long-acting topoisomerase I inhibitor for use in a method that is useful for (among other things) treating a patient suffering from a condition that is responsive to treatment with the compound. The method comprises administering a therapeutically effective amount of the given topoisomerase inhibitor. Other modes of administration are also contemplated, such as pulmonary, nasal, buccal, rectal, sublingual, transdermal, and parenteral. As used herein, the term "parenteral" includes subcutaneous, intravenous, intra-arterial, intraperitoneal, intracardiac, intrathecal, and intramuscular injection, as well as infusion injections.

The presently described method wherein a long-acting topoisomerase II inhibitor and a long-acting topoisomerase I inhibitor are administered to a patient may be used to treat any condition that can be remedied or prevented by this approach. Exemplary conditions are cancers, such as, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell cancer, basal cell cancer, adenocarcinoma, sweat gland cancer, sebaceous gland cancer, papillary cancer, papillary adenocarcinomas, cystadenocarcinoma, medullary cancer, bronchogenic cancer, renal cell cancer, hepatoma, bile duct cancer, choriocarcinoma, seminoma, embryonal cancer, Wilms' tumor, cervical cancer, testicular cancer, lung cancer, small cell lung cancer, bladder cancer, epithelial cancer, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma and leukemias.

## EXPERIMENTAL

### EXAMPLE 1

### Toxicology Study of Long Acting Topoisomerase I Inhibitor and Long Acting Topoisomerase II Inhibitor Combination Therapy in Rats

A pentaerythritol-based multi-arm polymer conjugate of irinotecan ("PBMAPCI," obtained from Nektar Therapeutics, San Francisco CA), a long-acting topoisomerase I inhibitor, and doxorubicin HCL liposome injection ("DLI," available under the DOXIL® brand from Janssen Biotech, Inc., Horsham PA), a long-acting topoisomerase II inhibitor, were administered to rats.

The objectives of the study were to assess the toxicity and associated toxicokinetic profile of PBMAPCI when administered in combination with DLI as two successive single 30-minute intravenous infusions in female Sprague Dawley rats, and to compare those to the toxicity and profiles of DLI alone following a 21-day observation period. A total of 119 female Sprague Dawley rats were assigned to five dose groups as described in Table 1.

**Table 1**

| Dosing Groups | | | | | | | | | |
|---|---|---|---|---|---|---|---|---|---|
| Group No. | Group Designation | Dose Level mg/kg ($mg/m^2$) | | Dose Concentration (mg/mL) | | Dose Rate (mL/kg/h) | Number of Female Animals | | |
| | | PBMAPCI | DLI | PBMAPCI | DLI | | Main | Hematology | TK |
| 1 | Saline Control* | 0 | 0 | 0 | 0 | 24/5 | 4 | 12 | 3 |
| 2 | DLI | 0** | 5 (30) | 0 | 2 | 24/5 | 4 | 12 | 9 |
| 3 | Low Dose PBMAPCI/DLI | 10 (60) | 5 (30) | 0.833 | 2 | 24/5 | 4 | 12 | 9 |
| 4 | Mid Dose PBMAPCI/DLI | 30 (180) | 5 (30) | 2.5 | 2 | 24/5 | 4 | 12 | 9 |
| 5 | High Dose PBMAPCI/DLI | 90 (540) | 5 (30) | 7.5 | 2 | 24/5 | 4 | 12 | 9 |

* The Saline Control animals received 0.9% sodium chloride for injection, USP over 2 sessions of 30 minutes.
** The DLI animals received the PBMAPCI vehicle (6.0 mg/mL lactic acid in 5% Dextrose in water for injection USP at pH 4.5±0.2) alone.
# Dose levels for PBMAPCI are expressed in terms of mg irinotecan equivalents; consequently, a correction factor of 10.204 was used when weighing and dispensing the test article powder.

Clinical observations, clinical chemistry, hematology, and histopathology were performed and toxicokinetic samples were collected for PBMAPCI, irinotecan, SN38, PEG-SN38, and doxorubicin levels. DLI-treated rats had depressed body weight gain by seven days post-dose administration and decreased white blood cell counts due to a decrease in lymphocytes compared to the concurrent vehicle control. These effects completely recovered by 21 days post dose. DLI-related splenic hypocellularity was observed 21 days after dosing. Of note, PBMAPCI at up to 540 mg/$m^2$ did not exacerbate the hematologic or body weight changes caused by DLI. PBMAPCI in combination with DLI (30 mg/$m^2$) increased the incidence and severity of splenic hypocellularity observed with 30 mg/$m^2$ PLD alone only at the highest dose of PBMAPCI at 540 mg/$m^2$. In conclusion, PBMAPCI does not exacerbate the hematologic effects (up to 540 mg/$m^2$) or splenic hypocellularity (up to 180 mg/$m^2$) caused by a single 30 mg/$m^2$ dose of DLI at SN38 levels that are more than 70- fold (based AUC at 180 mg/$m^2$) of that observed at PBMAPCI dose of 145 mg/kg in humans.

**EXAMPLE 2**

**Efficacy Study of Long Acting Topoisomerase I Inhibitor and Long Acting Topoisomerase II Inhibitor Combination Therapy in Nude Mice**

A pentaerythritol-based multi-arm polymer conjugate of irinotecan ("PBMAPCI"), a long-acting topoisomerase I inhibitor, was evaluated both alone and in combination with doxorubicin HCL liposome injection ("DLI," available under the DOXIL® brand from Janssen Biotech, Inc., Horsham PA), a long-acting topoisomerase II inhibitor, in the A2780 human ovarian carcinoma xenograft model in female nude mice.

PBMAPCI at 100 or 150 mg/kg (irinotecan equivalents) was administered intravenously for one dose. DLI at 5 or 10 mg/kg was administered intravenously weekly for two doses. Study control animals received one dose of D5W. Tumors were monitored and animals were euthanized when their tumors reached an endpoint volume of 2000 $mm^3$ or on Day 76, whichever came first, and the time-to-endpoint (TTE) was calculated for each mouse. Response was determined from an analysis of tumor growth delay (TGD), defined as the change in median TTE in treated versus control mice and from statistical assessment of differences in survival. Median tumor growth, tumor regression, and treatment tolerability also were considered. The results of the A2780 TGD study are presented in this example.

Female athymic nude mice (*nu/nu,* Harlan) were 9 to 10 weeks old, with body weights ranging from 18.1 to 27.5 g, at the beginning of the study. The animals were fed *ad libitum* water (reverse osmosis, 1 ppm Cl) and NIH 31 Modified and Irradiated Lab Diet® consisting of 18.0% crude protein, 5.0% crude fat, and 5.0% crude fiber. The test animals were housed on irradiated Enrich-o'cobs™ Laboratory Animal Bedding in static microisolators on a 12-hour light cycle at 20-22°C (68-72°F) and at 40-60% humidity.

A2780 tumor cells were cultured in RPMI-1640 medium containing 10% fetal bovine serum, 0.3 units/mL insulin, and 2 mM glutamine, 100 units/mL penicillin G, 100 μg/mL streptomycin sulfate and 25 μg/mL gentamicin. The cells were cultured in tissue culture flasks in a humidified incubator at 37 °C, in an atmosphere of 5% $CO_2$ and 95% air.

On the day of implant, cultured A2780 tumor cells were harvested during log phase growth and re-suspended in phosphate-buffered saline at a concentration of 5 x $10^7$ cells/mL. Xenografts were initiated by subcutaneously implanting 1 x $10^7$ A2780 tumor cells (0.2 mL suspension) into the right flank of each test animal and tumors were monitored as their volumes approached 80-120 $mm^3$. Nine days later, designated as Day 1 of the study, mice were sorted into treatment groups with individual tumor volumes ranging from 75 to 144 $mm^3$ and group mean tumor volumes of 106 to 108 $mm^3$. Volume was calculated using the formula:

$$\text{Tumor Volume}(\text{mm}^3) = \frac{w^2 \times l}{2}$$

where w = width and 1 = length in mm of a A2780 tumor. Tumor weight may be estimated with the assumption that 1 mg is equivalent to 1 $mm^3$ of tumor volume.

PBMAPCI was obtained as a dry powder in an amber vial that was stored at -20°C. PBMAPCI dosing solutions were formulated at 80.645 and 53.762 mg/mL on each day of dosing in 5% dextrose in de-ionized water (D5W).

DLI (obtained commercially, Lot No. 1015134, 2 mg/mL) dosing solutions were formulated weekly at 1 and 0.5 mg/mL in D5W. Dosing solutions were stored at 4°C until use.

On Day 1, test animals were sorted into nine groups (n = 10) and treatment was initiated. Doses of PBMAPCI are expressed as equivalent doses of irinotecan (1075.24 mg/kg PBMAPCI =100 mg/kg irinotecan equivalents and 1612.90 mg/kg PBMAPCI = 150 mg/kg irinotecan equivalents). Animals in the study control group, Group 1, received D5W, intravenously (i.v.), for one dose (qd x1). Animals in Groups 2 and 3 received PBMAPCI at 100 or 150 mg/kg, i.v., qd x1, respectively. Animals in Groups 4 and 5 received DLI at 5 or 10 mg/kg, i.v., weekly for two doses (qwk x2), respectively. Animals in Groups 6 and 7 received PBMAPCI at 100 mg/kg, i.v., qd x1, in combination with DLI at 5 or

10 mg/kg, i.v., qwk x2, respectively. Animals in Groups 8 and 9 received PBMAPCI at 150 mg/kg, i.v., qd x1, in combination with DLI at 5 or 10 mg/kg, i.v., qwk x2, respectively. D5W and PBMAPCI were dosed at 20 mL/kg (0.4 mL/20 g mouse) scaled to the body weight of each animal. DLI was dosed at 10 mL/kg (0.2 mL/20 g mouse) scaled to the body weight of each animal.

Tumors were measured using calipers twice weekly. Each animal was euthanized when its neoplasm reached the predetermined endpoint volume (2000 $mm^3$) or at the end of the study, whichever came first. The study was extended from 45 to 76 days to follow responders. The time to endpoint (TTE) for each test animal was calculated by the following equation:

$$\mathrm{TTE} = \frac{\log_{10}(\text{endpoint volume}) - b}{m}$$

where TTE is expressed in days, endpoint volume is expressed in $mm^3$, b is the intercept, and m is the slope of the line obtained by linear regression of a log-transformed tumor growth data set. The data set is comprised of the first observation that exceeded the endpoint volume used in analysis and the three consecutive observations that immediately preceded the attainment of this endpoint volume. Animals that did not reach the endpoint volume were assigned a TTE value equal to the last day of the study (Day 76). An animal classified as having died from treatment-related (TR) causes or non-treatment related metastasis (NTRm) causes was assigned a TTE value equal to the day of death. An animal classified as having died from non-treatment-related (NTR) causes was excluded from TTE calculations.

Treatment efficacy was determined from tumor growth delay (TGD), which is defined as the increase in the median TTE for a treatment group compared to the control group:

$$\mathrm{TGD} = \mathrm{T} - \mathrm{C},$$

expressed in days, or as a percentage of the median TTE of the control group:

$$\%\mathrm{TGD} = \frac{\mathrm{T} - \mathrm{C}}{\mathrm{C}} \times 100$$

where:

T = median TTE for a treatment group, and
C = median TTE for control Group 1.

Treatment efficacy was also determined from the incidence, magnitude, and durability of regression responses observed during the study. The MTV(n) is defined as the median tumor volume on the day of TGD analysis in the number of animals remaining, n, whose tumors have not attained the endpoint volume.

Treatment may cause partial regression (PR) or complete regression (CR) of the tumor in an animal. In a PR response, the tumor volume is 50% or less of its Day 1 volume for three consecutive measurements during the course of the study, and equal to or greater than 13.5 $mm^3$ for one or more of these three measurements. In a CR response, the tumor volume is less than 13.5 $mm^3$ for three consecutive measurements during the course of the study. An animal with a CR response at the termination of a study is additionally classified as a tumor-free survivor (CR/TFS). Tumor regressions were monitored and recorded.

Animals were weighed daily on Days 1-5 then twice weekly until the completion of the study. Test animals were observed frequently for overt signs of any adverse, treatment-related side effects, and clinical signs of toxicity were recorded when observed. Acceptable toxicity for the maximum tolerated dose (MTD) was defined as a group mean body weight loss of less than 20% during the test, and not more than 10% mortality due to TR deaths. A death was classified as TR if it was attributable to treatment side effects as evidenced by clinical signs and/or necropsy, or due to unknown causes during the dosing period or within 14 days of the last dose. A death was classified as NTR if there is no evidence that the death was related to treatment side effects. NTR deaths may be further characterized based on cause of death. A death was classified as NTRa if it resulted from an accident or human error. A death was classified as NTRm if necropsy indicated that it may have resulted from tumor dissemination by invasion and/or metastasis. A death was classified as NTRu if the death was of unknown etiology or the cause of death is not known and no evidence of death related to treatment side effects, metastasis, accident or human error is available, although death due to treatment side effects cannot be excluded.

Prism (GraphPad) for Windows 3.03 was used for all statistical analysis and graphical presentations. The logrank test was employed to assess the significance of the difference between the overall survival experiences of two groups. The logrank test analyzed the individual TTEs for all animals in a group, except those lost to the study due to NTR deaths. The two-tailed statistical analysis was conducted at $P = 0.05$. Prism reports results as non-significant (ns) at $P > 0.05$, significant (symbolized by "*") at $0.01 < P \leq 0.05$, very significant ("**") at $0.001 < P \leq 0.01$ and extremely significant ("***") at $P \leq 0.001$. Since the logrank test is a test of significance and does not provide an estimate of the size of the difference between groups, all levels of significance are reported as either significant or non-significant within the context of this report.

Individual TTEs for all animals in each group are presented as a scattergram in **FIG. 1.** Tumor growth curves, **FIG. 2** (upper panel), show each group's median tumor volume as a function of time. When an animal exited the study due to tumor size or TR death, the final tumor volume recorded for the animal was included with the data used to calculate the median volume at subsequent time points. Tumor growth curves were truncated when tumors in more than 50% of the assessable animals in a group attained endpoint or when treatment exceeded the limit for acceptable toxicity for the MTD. Kaplan-Meier plots, **FIG. 2** (lower panel), show the percentage of animals remaining in the study versus time using the same TTE data as the scattergram in **FIG. 1.** The percent group mean body weight changes from Day 1 with standard error of the mean (SEM) are presented in **FIG. 3.**

Test animals in the study were treated in accordance with the protocol in Table 2.

**Table 2**

| Protocol Design for A2780 Human Ovarian Carcinoma Study in Mice | | | | | | | | | |
|---|---|---|---|---|---|---|---|---|---|
| | | Treatment Regimen 1 | | | | Treatment Regimen 2 | | | |
| Group | n | Agent | mg/kg | Route | Schedule | Agent | mg/kg | Route | Schedule |
| 1* | 10 | D5W | - | iv | qd x 1 | - | - | - | - |
| 2 | 10 | MP PBMAPCI | 100 | iv | qd x 1 | - | - | - | - |
| 3 | 10 | MP PBMAPCI | 150 | iv | qd x 1 | - | - | - | - |
| 4 | 10 | Doxil DLI | 5 | iv | qwk x 2 | - | - | - | - |
| 5 | 10 | Doxil DLI | 10 | iv | qwk x 2 | - | - | - | - |
| 6 | 10 | MP PBMAPCI | 100 | iv | qd x 1 | Doxil DLI | 5 | iv | qwk x 2 |
| 7 | 10 | MP PBMAPCI | 100 | iv | qd x 1 | Doxil DLI | 10 | iv | qwk x 2 |
| 8 | 10 | MP PBMAPCI | 150 | iv | qd x 1 | Doxil DLI | 5 | iv | qwk x 2 |
| 9 | 10 | MP PBMAPCI | 150 | iv | qd x 1 | Doxil DLI | 10 | iv | qwk x 2 |
| *Key: # = control group; PBMAPCI expressed as equivalent doses of irinotecan (1075.24 mg/kg PBMAPCI = 100 mg/kg irinotecan equivalents and 1612.90 mg/kg PBMAPCI = 150 mg/kg irinotecan equivalents | | | | | | | | | |

The study was terminated on Day 76. Table 3 summarizes the treatment responses for each group including the statistical outcomes as presented below.

## Table 3
## Summary of Treatment Responses for A2780 Human Ovarian Carcinoma Study in Mice

| | | Treatment Regimen | | | | Median | | | Statistical Significance | | | | | MTV (n) | Regressions | | | Mean BW | Deaths | |
|---|---|---|---|---|---|---|---|---|---|---|---|---|---|---|---|---|---|---|---|---|
| Group | n | Agent | mg/kg | Route | Schedule | TTE | T-C | %TGD | vs G1 | vs G2 | vs G3 | vs G4 | vs G5 | Day 76 | PR | CR | TFS | Nadir | TR | NTR |
| 1# | 10 | vehicle | --- | iv | qd x 1 | 12.0 | --- | --- | --- | --- | --- | --- | --- | --- | 0 | 0 | 0 | --- | 0 | 0 |
| 2 | 10 | MI PBMAPCI | | iv | qd x 1 | 43.8 | 31.8 | 265 | *** | --- | --- | --- | --- | 0 (3) | 0 | 10 | 3 | -0.5% Day 2 | 0 | 0 |
| 3 | 10 | MI | | iv | qd x 1 | 49.6 | 37.6 | 313 | *** | --- | --- | --- | --- | 0 (1) | 0 | 10 | 1 | -0.1% Day 4 | 0 | 0 |
| 4 | 10 | Dox DLI | | iv | qwk x 2 | 19.4 | 7.4 | 62 | *** | --- | --- | --- | --- | --- | 0 | 0 | 0 | -0.6% Day 9 | 0 | 0 |
| 5 | 9 | Dox | | iv | qwk x 2 | 36.1 | 24.1 | 201 | *** | --- | --- | --- | --- | --- | 1 | 0 | 0 | -8.6% Day 13 | 0 | 1 |
| 6 | 10 | MF<br>Dox | | iv<br>iv | qd x 1<br>qwk x 2 | 76.0 | 64.0 | 533 | *** | ** | --- | *** | --- | 0 (10) | 0 | 10 | 9 | -4.9% Day 3 | 0 | 0 |
| 7 | 10 | MF<br>Dox PBMAPCI | | iv<br>iv | qd x 1<br>qwk x 2 | 76.0 | 64.0 | 533 | *** | ** | --- | --- | *** | 0 (10) | 0 | 10 | 10 | -9.8% Day 76 | 0 | 0 |
| 8 | 10 | MF DLI<br>Dox | | iv<br>iv | qd x 1<br>qwk x 2 | 76.0 | 64.0 | 533 | *** | --- | *** | *** | --- | 0 (8) | 0 | 10 | 8 | -4.2% Day 9 | 0 | 0 |
| 9 | 10 | MF<br>Dox | | iv<br>iv | qd x 1<br>qwk x 2 | 76.0 | 64.0 | 533 | *** | --- | *** | --- | *** | 0 (10) | 0 | 10 | 10 | -6.7% Day 9 | 0 | 0 |

*Key: # = control group; PBMAPCI expressed as equivalent doses of irinotecan (1075.24 mg/kg PBMAPCI = 100 mg/kg irinotecan equivalents and 1612.90 mg/kg PBMAPCI = 150 mg/kg irinotecan equivalents.
Study endpoint = 2000 $mm^3$, total days - 76, n = number of animals in a group not dead from accidental or unknown causes, or euthanized for sampling, MTV(n) = median tumor volume ($mm^3$) for the number of animals on the day of TGD analysis (excludes animals with tumor volume at endpoint), TTE = time-to-endpoint, TGD = T-C = difference between median TTE (days) of treated verses designated control group, %TGD = [(T-C)/C] x 100, maxiumum TGD - 64.0 days (544%). Statistical significance - Logrank test: ne = not evaluable, ns = non-significant, * = $0.01 < P \leq 0.05$, ** = $0.001 < P \leq 0.01$, *** = $P \leq 0.001$ when compared to group indicated, PR = partial regressions, CR = total number complete regressions, TFS = tumor-free survivors, i.e., CRs at end of study, Mean BW nadir = lowest group mean body weight, as % change from Day 1, "---" indicates no decrease in mean body weight was observed, TR = treatment-related death, NTR = non-treatment-related death.

Animals in Group 1 received D5W and were designated as the control group for calculating TGD and statistical significance by the logrank test. As shown in **FIG. 1,** ten tumors in control mice grew to endpoint without regressions between 10.6 and 13.9 days. As presented in Table 2, the median TTE for control mice was 12.0 days, establishing a maximum possible TGD of 64.0-days (533% TGD) for this study. Median tumor growth progressed rapidly, as seen in **FIG. 2,** upper panel.

Animals in Groups 2 and 3 received a single dose of PBMAPCI at 100 or 150 mg/kg, respectively. Seven tumors in Group 2 test mice grew to endpoint between 31.7 and 49.9 days and three tumor free animals survived the study. Nine tumors in Group 3 test mice grew to endpoint between 36.0 and 60.6 days and one tumor free animal survived the study. The treatment outcomes yielded respective median TTEs of 43.8 days (31.8-day or 265% TGD) and 49.6 days (37.6-day or 313% TGD), significance versus control ($P$ < 0.001), and ten CRs each including the aforementioned four TFS. Median tumor burdens in Groups 2 and 3 decreased to below the limit of palpability on Day 9 where they remained until Day 20 or 23, respectively, followed by tumor rebound.

Animals in Groups 4 and 5 received DLI at 5 or 10 mg/kg, respectively. Ten tumors in Group 4 test mice grew to endpoint without regressions between 17.7 and 34.1 days. One Group 5 animal, missing from its assigned microisolator on Day 41, was classified as an NTRu death and was censored from analysis. Tumors in the remaining nine Group 5 test mice grew to endpoint between 28.8 and 57.3 days. The treatment outcomes yielded respective median TTEs of 19.4 days (7.4-day or 62% TGD) and 36.1 days (24.1-day or 201% TGD), significance versus control ($P$ < 0.001), and one PR in a Group 5 animal. Median tumor growth was measurably delayed in Group 4 mice and considerably delayed in Group 5 mice.

Animals in Groups 6-9 received PBMAPCI at 100 or 150 mg/kg in combination with DLI at 5 or 10 mg/kg, respectively. Ten test animals each in Groups 6, 7, and 9, and eight in Group 8 survived the study. Two tumors in Group 8 test mice attained calculated endpoints of 71.6 and 75.0 days. The treatment outcomes yielded the maximum attainable median TTE of 76.0 days (64.0-day or 533% TGD), significance versus control ($P$ < 0.001), and ten CRs each. The ten CRs in Groups 6-9 were further classified as nine, ten, eight, and ten TFSs, respectively. The differences in overall survival between each combination therapy group and its corresponding PBMAPCI and DLI monotherapies were significant ($P$ < 0.01, Group 6 or 7 vs. Group 2, $P$ < 0.001, all others). Median tumor burdens decreased to below the limit of palpability on Day 9 where they remained.

Animals were monitored for signs of treatment-related (TR) side effects and for changes in body weight (Table 2 and **FIG. 3**). No TR deaths were recorded. In Groups 5-9 treatments with 10 mg/kg DLI alone or DLI combined with PBMAPCI caused moderate group mean body weight losses that rebounded after dosing was completed. In Group 7, additional mean body losses occurred after Day 69. In Group 6, clinical observations on Day 69 described four animals as thin and hunched accompanied by weight loss.

The 76-day TGD study evaluated two single dose regimens of NKTR-102 (PBMAPCI), alone and combined with DLI, in the A2780 human ovarian carcinoma xenograft model in female nude mice. Tumors in control mice grew uniformly to endpoint without regressions, yielding a median TTE of 12.0 days and a maximum possible TGD of 64.0-days (533% TGD). All treatment regimens attained significance versus control.

The A2780 model responded well to a single dose of PBMAPCI at 100 or 150 mg/kg, yielding respective TGDs of 265% and 313% and ten CRs each with four further classified as TFSs. Median tumor burdens decreased to below the limit of palpability for 12-13 days followed by tumor rebound.

DLI monotherapies at 5 or 10 mg/kg yielded dose related TGDs of 62% and 201% TGD and one PR in an animal that received 10 mg/kg DLI. Median tumor growth also exhibited dose related delays but neither group exhibited a period of progression free survival.

The A2780 model responded strongly to PBMAPCI combined with DLI yielding the maximum attainable TGD of 533% and 80-100% tumor free survivors. The differences in overall survival between each combination therapy group and the corresponding PBMAPCI and DLI monotherapies were significant. Median tumor burdens decreased to below the limit of palpability on Day 9 where they remained until study end. These exceptional responses did not allow differences among the four combination therapies to be ascertained.

No TR deaths were recorded. Treatments with 10 mg/kg DLI alone or DLI combined with PBMAPCI were acceptably tolerated, causing moderate mean body weight losses during dosing followed by weight rebound. All other treatments were well-tolerated.

In summary, PBMAPCI and DLI, alone and combined, were significantly active in the A2780 model. PBMAPCI monotherapies yielded sizeable TGDs, intervals of progression free survival, and complete regressions in all animals, including four that were tumor free at study end. Outcomes for DLI alone were dose related and exhibited slowed disease progression. Responses to PBMAPCI combined with DLI were exceptionally strong, yielding the maximum attainable TGD and 80-100% tumor free survivors. Treatments with 10 mg/kg DLI alone or DLI combined with PBMAPCI were acceptably tolerated. All other treatments were well-tolerated.

In summary, PBMAPCI and DLI, alone and combined, were significantly active in the A2780 model.

## EXAMPLE 3

### Combination Study in Humans

A pentaerythritol-based multi-arm polymer conjugate of irinotecan ("PBMAPCI"), a long-acting topoisomerase I inhibitor, and doxorubicin HCL liposome injection ("DLI," available under the DOXIL® brand from Janssen Biotech, Inc., Horsham PA), a long-acting topoisomerase II inhibitor, are administered to humans. In this dose-escalation study, the safety and tolerability of PBMAPCI when given on an every 28 day schedule in combination with DLI to humans with advanced/metastatic refractory solid tumors is evaluated. Unless otherwise indicated by toxicity, the dose for DLI is given at a fixed dose while the PBMAPCI dose is to be escalated.

In this study, patients are grouped into cohorts of three to six patients suffering from advanced and/or metastatic refractory solid tumors until the maximum-tolerated dose is found using a 3+3 dose escalation scheme as described in Table 4.

**Table 4**

| Dose-Escalation* Protcol | | |
|---|---|---|
| **Cohort** | **PBMAPCI ($mg/m^2$)** | **DLI ($mg/m^2$)** |
| 1 | 80 | 40 |
| 2 | 120 | 40 |
| 3 | 145 | 40 |
| 4 | 170 | 40 |
| * Dosages to be adjusted downward in the event to toxicity. | | |

One cycle of therapy is defined as 28 days. For Cycle 1, one dose of DLI is administered IV over 60 ± 10 minutes on Day 1 of Cycle 1, followed by PBMAPCI administered IV over 90 ± 10 minutes on Day 8 of Cycle 1. For Cycle 2 and beyond, PBMAPCI is administered first as an IV infusion over 90 ± 10 minutes on Day 1 of each cycle followed immediately by DLI administered IV over 60 ± 10 minutes. Once the maximum-tolerated dose is identified, the study may be expanded and up to 15 additional patients.

Upon completion of the study, pharmacokinetic data is obtained and it is concluded that the combination of PBMAPCI and DLI evidences a favorable tumor response rate

## Claims

1. A long-acting topoisomerase I inhibitor for use in a method comprising the steps of:

   (a) administering to a patient a topoisomerase II-inhibiting amount of a long-acting topoisomerase II inhibitor; and (b) administering to the patient a topoisomerase I-inhibiting amount of a long-acting topoisomerase I inhibitor, wherein the long-acting topoisomerase I inhibitor encompasses the following structure

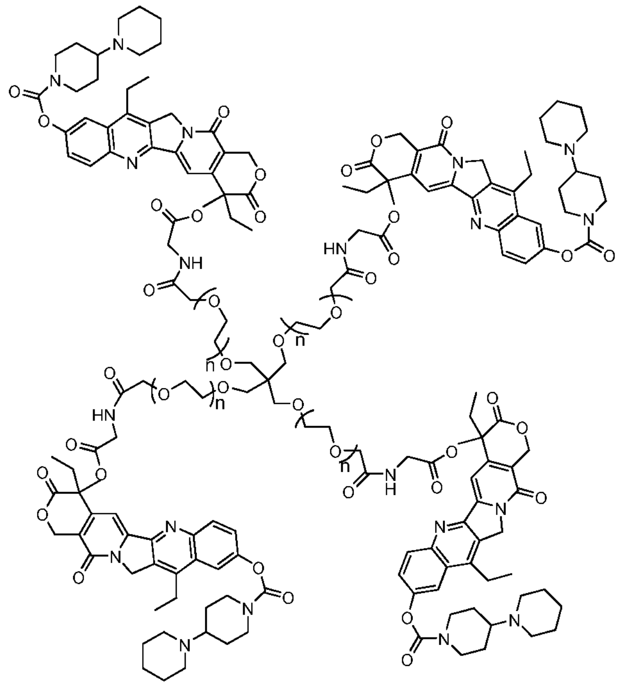

   wherein each n is an integer ranging from 40 to 500, and pharmaceutically acceptable salts thereof, and wherein the long-acting topoisomerase II inhibitor is a topoisomerase II inhibitor which is released from its formulation over at least ten days.

2. The long-acting topoisomerase I inhibitor for use according to claim 1, wherein the patient is suffering from a cancer.

3. The long-acting topoisomerase I inhibitor for use according to claim 2, wherein the cancer is a solid cancer.

4. The long-acting topoisomerase I inhibitor for use according to claim 3, wherein the solid cancer is selected from the group consisting of ovarian cancer, breast cancer, colon cancer, colorectal cancer, gastric cancer, malignant melanoma, liver cancer, small cell lung cancer, non-small cell lung cancer, thyroid cancers, kidney cancer, cancer of the bile duct, brain cancer, cervical cancer, maxillary sinus cancer, bladder cancer, esophageal cancer, Hodgkin's disease and adrenocortical cancer.

5. The long-acting topoisomerase I inhibitor for use according to claim 2, wherein the cancer is ovarian cancer.

6. The long-acting topoisomerase I inhibitor for use according to claim 5, wherein the ovarian cancer is platinum-resistant ovarian cancer.

**7.** The long-acting topoisomerase I inhibitor for use according to claim 2, wherein the cancer is colorectal cancer.

**8.** The long-acting topoisomerase I inhibitor for use according to claim 2, is breast cancer.

**9.** The long-acting topoisomerase I inhibitor for use according to claim 2, wherein the cancer is selected from group consisting of lymphoma cancers, leukemia cancers, rhabdomyosarcoma and neuroblastoma.

**10.** The long-acting topoisomerase I inhibitor for use according to claim 1, wherein the patient is human.

**11.** The long-acting topoisomerase I inhibitor for use according to claim 1, wherein step (a) is carried out prior to step (b) being carried out.

**12.** The long-acting topoisomerase I inhibitor for use according to claim 1, wherein (a) is carried out after step (b) is carried out.

**13.** The long-acting topoisomerase I inhibitor for use according to claim 1, wherein steps (a) and (b) are carried out simultaneously.

## Patentansprüche

**1.** Langzeit-Topoisomerase I-Inhibitor zur Verwendung in einem Verfahren umfassend die Schritte:

(a) Verabreichen an einen Patienten eine Topoisomerase II inhibierende Menge eines Langzeit-Topoisomerase II-Inhibitors und (b) Verabreichen an den Patienten eine Topoisomerase I inhibierende Menge eines Langzeit-Topoisomerase I-Inhibitors, wobei der Langzeit-Topoisomerase I-Inhibitor die folgende Struktur umfasst

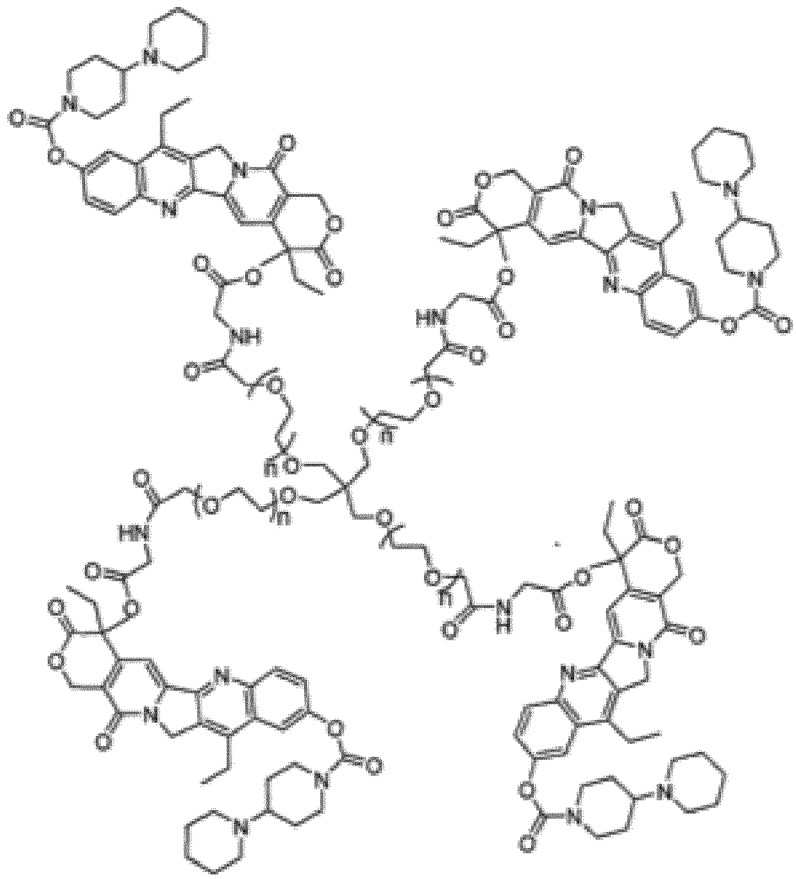

wobei jedes n eine ganze Zahl ist, die von 40 bis 500 reicht, und pharmazeutisch annehmbare Salze davon, und wobei der Langzeit-Topoisomerase II-Inhibitor ein Topoisomerase II-Inhibitor ist, der aus seiner Formulierung

über mindestens zehn Tage freigesetzt wurde.

2. Langzeit-Topoisomerase I-Inhibitor zur Verwendung nach Anspruch 1, wobei der Patient an Krebs leidet.

3. Langzeit-Topoisomerase I-Inhibitor zur Verwendung nach Anspruch 2, wobei der Krebs ein solider Krebs ist.

4. Langzeit-Topoisomerase I-Inhibitor zur Verwendung nach Anspruch 3, wobei der solide Krebs ausgewählt ist aus der Gruppe bestehend aus Eierstockkrebs, Brustkrebs, Darmkrebs, Kolorektalkrebs, Magenkrebs, malignem Melanom, Leberkrebs, kleinzelligem Lungenkrebs, nicht-kleinzelligem Lungenkrebs, Schilddrüsenkrebsarten, Nierenkrebs, Gallengangkrebs, Hirntumor, Gebärmutterhalskrebs, Kieferhöhlenkrebs, Blasenkrebs, Speiseröhrenkrebs, Morbus Hodgkin und Nebennierenkrebs.

5. Langzeit-Topoisomerase I-Inhibitor zur Verwendung nach Anspruch 2, wobei der Krebs Eierstockkrebs ist.

6. Langzeit-Topoisomerase I-Inhibitor zur Verwendung nach Anspruch 5, wobei der Eierstockkrebs Platin-resistenter Eierstockkrebs ist.

7. Langzeit-Topoisomerase I-Inhibitor zur Verwendung nach Anspruch 2, wobei der Krebs Kolorektalkrebs ist.

8. Langzeit-Topoisomerase I-Inhibitor zur Verwendung nach Anspruch 2, bei dem es sich um Brustkrebs handelt.

9. Langzeit-Topoisomerase I-Inhibitor zur Verwendung nach Anspruch 2, wobei der Krebs ausgewählt ist aus der Gruppe bestehend aus Lymphomkrebsarten, Leukämiekrebsarten, Rhabdomyosarkom und Neuroblastom.

10. Langzeit-Topoisomerase I-Inhibitor zur Verwendung nach Anspruch 1, wobei der Patient ein Mensch ist.

11. Langzeit-Topoisomerase I-Inhibitor zur Verwendung nach Anspruch 1, wobei Schritt (a) durchgeführt wird, bevor Schritt (b) durchgeführt wird.

12. Langzeit-Topoisomerase I-Inhibitor zur Verwendung nach Anspruch 1, wobei (a) durchgeführt wird, nachdem Schritt (b) durchgeführt wurde.

13. Langzeit-Topoisomerase I-Inhibitor zur Verwendung nach Anspruch 1, wobei die Schritte (a) und (b) gleichzeitig durchgeführt werden.

## Revendications

1. Inhibiteur de topoisomérase I à action prolongée destiné à être utilisé dans un procédé comprenant les étapes qui consistent à : (a) administrer à un patient une quantité inhibitrice de topoisomérase II d'un inhibiteur de topoisomérase II à action prolongée ; et (b) administrer au patient une quantité inhibitrice de topoisomérase I d'un inhibiteur de topoisomérase I à action prolongée, où l'inhibiteur de topoisomérase I à action prolongée contient la structure suivante

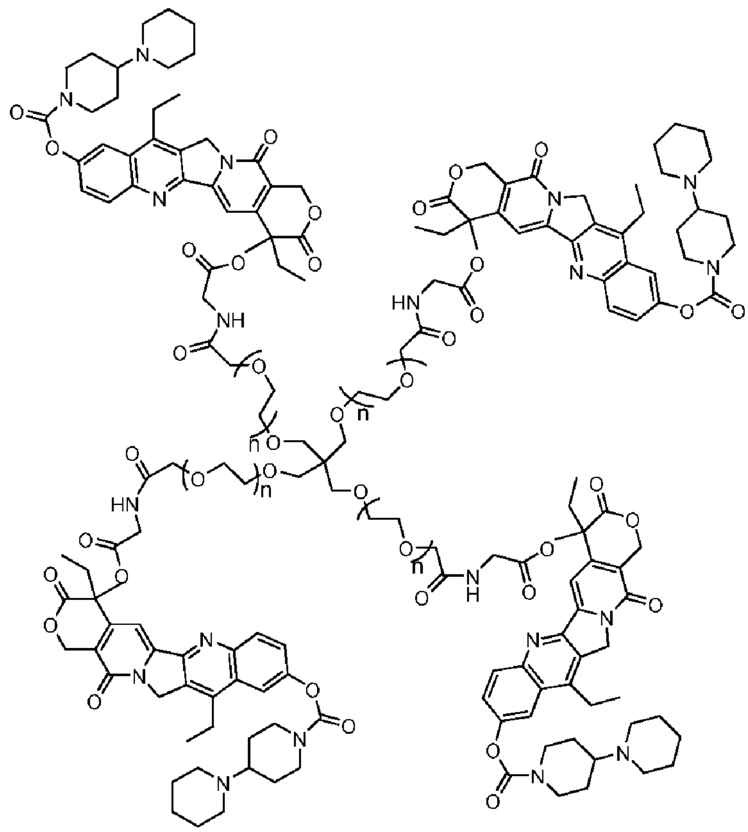

dans laquelle chaque n est un nombre entier allant de 40 à 500, et des sels pharmaceutiquement acceptables de celui-ci, et
où l'inhibiteur de topoisomérase II à action prolongée est un inhibiteur de topoisomérase II qui est libéré de sa formulation pendant au moins dix jours.

2. Inhibiteur de topoisomérase I à action prolongée destiné à être utilisé selon la revendication 1, le patient souffrant d'un cancer.

3. Inhibiteur de topoisomérase I à action prolongée destiné à être utilisé selon la revendication 2, le cancer étant un cancer solide.

4. Inhibiteur de topoisomérase I à action prolongée destiné à être utilisé selon la revendication 3, le cancer solide étant choisi dans le groupe consistant en le cancer de l'ovaire, le cancer du sein, le cancer du côlon, le cancer colorectal, le cancer gastrique, le mélanome malin, le cancer du foie, le cancer du poumon à petites cellules, le cancer du poumon non à petites cellules, les cancers de la thyroïde, le cancer du rein, le cancer des voies biliaires, le cancer du cerveau, le cancer du col de l'utérus, le cancer du sinus maxillaire, le cancer de la vessie, le cancer de l'oesophage, la maladie de Hodgkin et le cancer corticosurrénal.

5. Inhibiteur de topoisomérase I à action prolongée destiné à être utilisé selon la revendication 2, le cancer étant le cancer de l'ovaire.

6. Inhibiteur de topoisomérase I à action prolongée destiné à être utilisé selon la revendication 5, le cancer de l'ovaire étant un cancer de l'ovaire résistant au platine.

7. Inhibiteur de topoisomérase I à action prolongée destiné à être utilisé selon la revendication 2, le cancer étant le cancer colorectal.

8. Inhibiteur de topoisomérase I à action prolongée destiné à être utilisé selon la revendication 2, le cancer étant le cancer du sein.

9. Inhibiteur de topoisomérase I à action prolongée destiné à être utilisé selon la revendication 2, le cancer étant choisi dans le groupe consistant en les cancers de type lymphome, les cancers de type leucémie, le rhabdomyosarcome et le neuroblastome.

10. Inhibiteur de topoisomérase I à action prolongée destiné à être utilisé selon la revendication 1, le patient étant un être humain.

11. Inhibiteur de topoisomérase I à action prolongée destiné à être utilisé selon la revendication 1, dans lequel l'étape (a) est réalisée avant la réalisation de l'étape (b).

12. Inhibiteur de topoisomérase I à action prolongée destiné à être utilisé selon la revendication 1, dans lequel (a) est réalisée après que l'étape (b) est réalisée.

13. Inhibiteur de topoisomérase I à action prolongée destiné à être utilisé selon la revendication 1, dans lequel les étapes (a) et (b) sont réalisées simultanément.

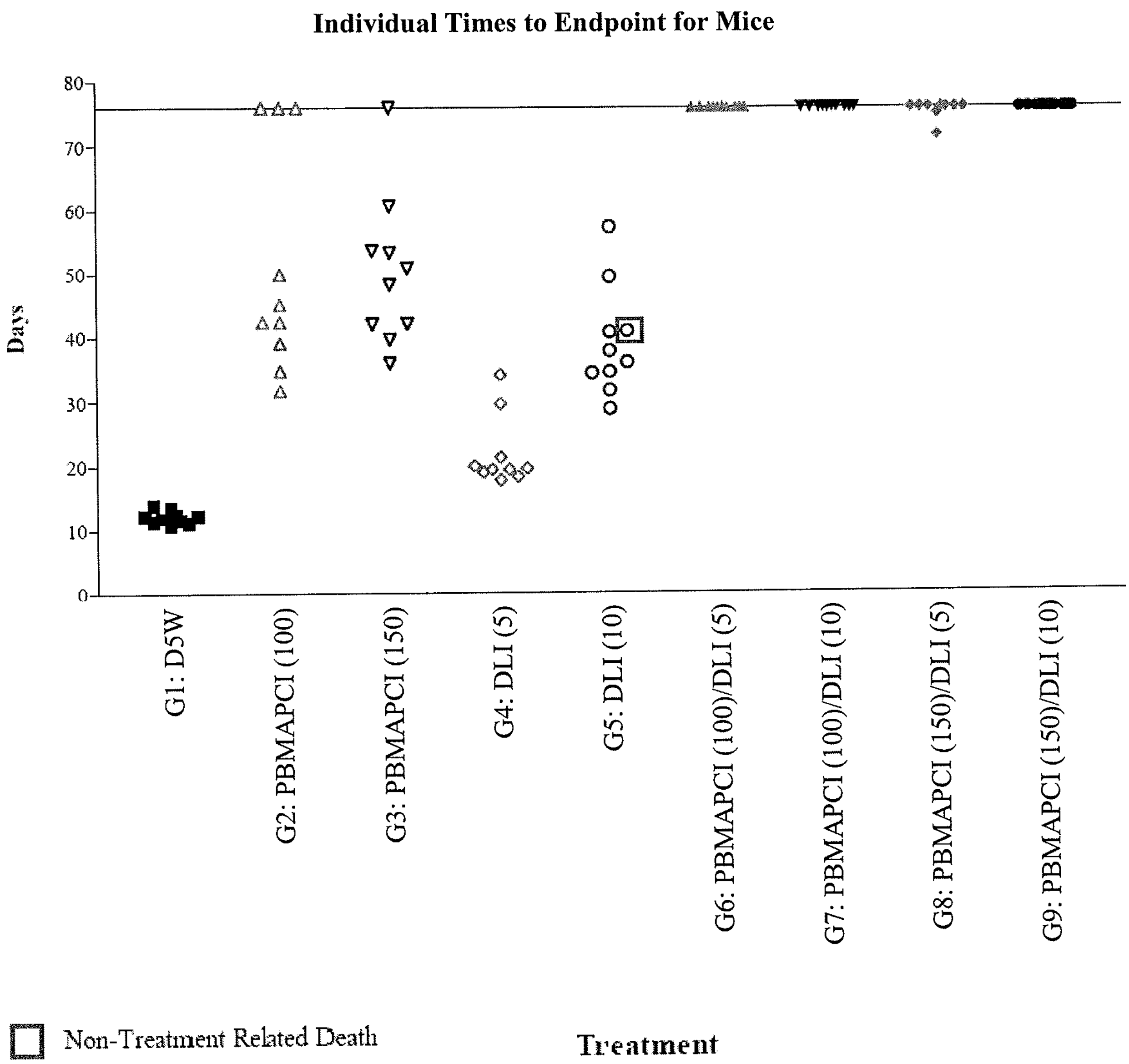
Individual Times to Endpoint for Mice
80
70
60
50
40
30
20
10
0
Days
G1: D5W
G2: PBMAPCI (100)
G3: PBMAPCI (150)
G4: DLI (5)
G5: DLI (10)
G6: PBMAPCI (100)/DLI (5)
G7: PBMAPCI (100)/DLI (10)
G8: PBMAPCI (150)/DLI (5)
G9: PBMAPCI (150)/DLI (10)
Non-Treatment Related Death
Treatment

**FIG. 1**

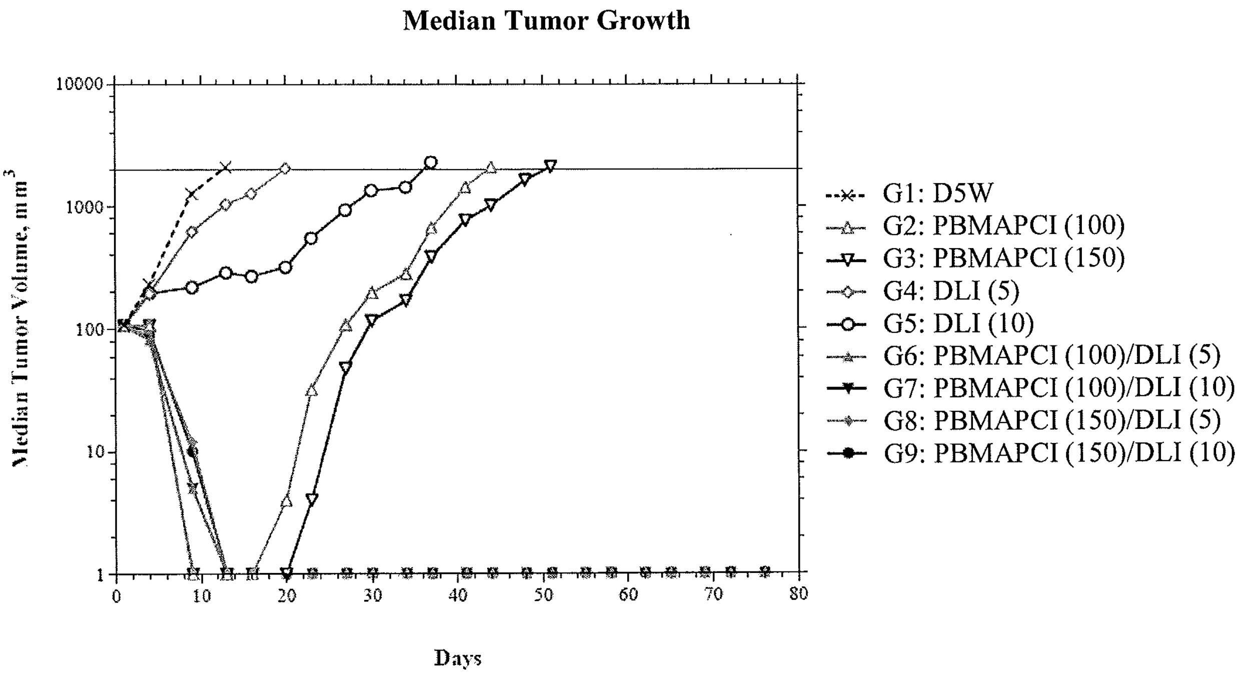
Median Tumor Growth
Median Tumor Volume, mm$^3$
Days
G1: D5W
G2: PBMAPCI (100)
G3: PBMAPCI (150)
G4: DLI (5)
G5: DLI (10)
G6: PBMAPCI (100)/DLI (5)
G7: PBMAPCI (100)/DLI (10)
G8: PBMAPCI (150)/DLI (5)
G9: PBMAPCI (150)/DLI (10)

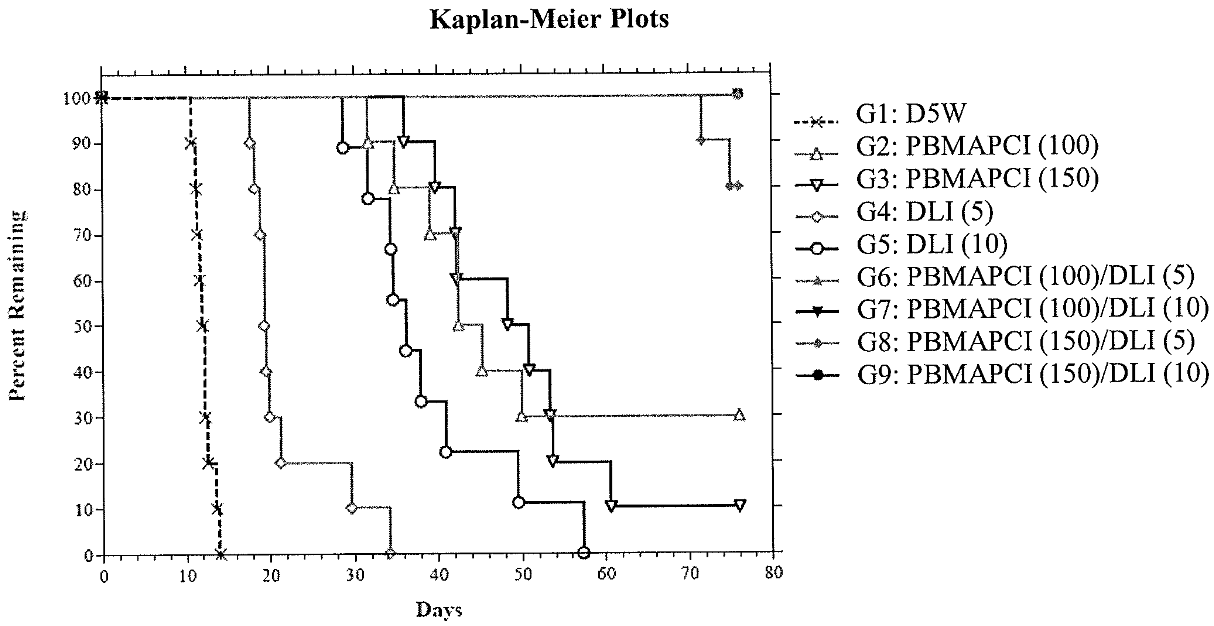
Kaplan-Meier Plots
Percent Remaining
Days
G1: D5W
G2: PBMAPCI (100)
G3: PBMAPCI (150)
G4: DLI (5)
G5: DLI (10)
G6: PBMAPCI (100)/DLI (5)
G7: PBMAPCI (100)/DLI (10)
G8: PBMAPCI (150)/DLI (5)
G9: PBMAPCI (150)/DLI (10)

FIG. 2

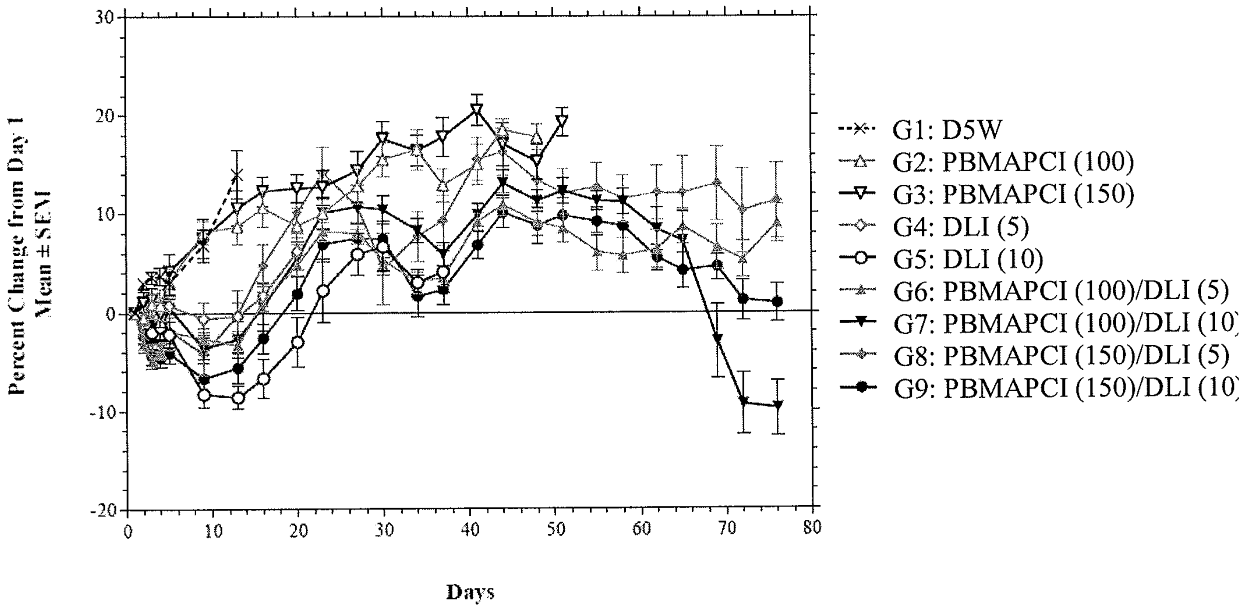
Percent Change from Day 1
Mean ± SEM
30
20
10
0
-10
-20
0
10
20
30
40
50
60
70
80
Days
G1: D5W
G2: PBMAPCI (100)
G3: PBMAPCI (150)
G4: DLI (5)
G5: DLI (10)
G6: PBMAPCI (100)/DLI (5)
G7: PBMAPCI (100)/DLI (10)
G8: PBMAPCI (150)/DLI (5)
G9: PBMAPCI (150)/DLI (10)

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7744861 B

### Non-patent literature cited in the description


- **POMMIER et al.** *Biochim. Biophys. Acta,* 1998, vol. 1400 (1-3), 83-105
- **WANG.** *Annu. Rev. Biochem.,* 1996, vol. 65, 635-92
- **TURMAN et al.** *Biochem. Med. Metab. Biol.,* 1993, vol. 50 (2), 210-25
- **CODERONI et al.** *Int. J. Biochem.,* 1990, vol. 22 (7), 737-46
- **KAISERMAN et al.** *Biochemistry,* 1988, vol. 27 (9), 3216-22
- **SAMUELS et al.** *J. Biol. Chem.,* 1992, vol. 267 (16), 1156-62
- **PILLOT et al.** *Journal of Clinical Oncology,* 2006, vol. 24 (18S), 12023
- **KRAUZE et al.** *Neuro-oncology,* 01 October 2007, 393-404
- **KEHRER et al.** *Clin. Can. Res.,* 2000, vol. 6, 3451-3458
- **GARCIA et al.** *2011 American Society of Clinical Oncology Annual Meeting,* 2011
- **LAGINHA et al.** *Clin. Can. Res.,* 2005, vol. 11 (19), 6944-6949